# EUROPEAN PATENT APPLICATION

(11) **EP 1 593 398 A1**
(43) Date of publication of application: **09.11.2005**
(21) Application number: 05290989.2
(22) Date of filing: 09.05.2005
(51) Int. Cl.: A61L 9/12, A47L 15/42

(54) **Air freshening method for automatic washing machine**

(30) Priority: 07.05.2004 EP 04291185
(71) Applicant: UNILEVER N.V., 3013 AL Rotterdam (NL); UNILEVER PLC, London EC4P 4BQ (GB)
(72) Inventor: Oldenburg, Willem Unilever R&D Vlaardingen, AT Vlaardingen 3133 (NL); Bornauw, Kenneth, 75858 Paris Cedex 17 (FR)
(74) Representative: Ahner, Francis

(57) **Abstract**

The invention provides a method for inhibiting malodour generated from the interior of an automatic washing machine, in particular an automatic dishwasher, the machine having a wash chamber (3) closable by a hinged door (2), and the method comprising the step of positioning a freshening strip (7) on an interior surface (5) of the door (2) which is exposed to the flow of steam exiting from the wash chamber (3) as the door (2) is opened upon completion or interruption of a wash cycle.

The freshening strip (7) is suitably formed from a perfumed gel made from polyamide resin.

The invention enables the freshening of the air coming from the machine directly after the wash.

## Description

### Field of the Invention

The present invention relates to a method of freshening the air coming from an automatic washing machine, in particular an automatic dishwasher, directly after the wash.

### Background and Prior Art

Undesirable odours can build up in the interior of an automatic washing machine such as an automatic dishwasher due to the accumulation of soiled articles prior to operation of the wash.

To combat these odours, fragrances may be formulated into the dishwasher detergent which is used in the wash. A problem with this route is that automatic dishwasher detergents typically contain powerful chemically reactive species such as bleaching agents, which are difficult to combine with oxidation sensitive components such as fragrances. Therefore it is not generally possible to incorporate a level of fragrance which would be sufficient to overcome undesirable odours accumulated in the machine interior, especially since dilution of the fragrance during the wash means that very little residual fragrance remains after the wash in any event.

An alternative solution is to hang a fragrancing device inside the dishwasher. For example, W096/38638 describes a dispenser for dispensing fragrance in the form of a waxy cake into a dishwasher during operation. The dispenser has a main body portion forming a cavity into which the waxy cake is secured by means of retaining pins, and a hinged lockable front plate having elongated openings through which water can pass to dissolve and release fragrance. A problem with this type of device is that the fragrancing effect produced can be too pronounced, with the fragrance lingering significantly and adhering noticeably to the washed items, especially when the items are made of plastic, which tends to pick up residual fragrance. This is a particular problem in small households where items may be left to accumulate in the dishwasher for a prolonged period prior to operation of the wash. The fragrancing device may also interfere with the operation of the wash, for example by impeding the rotation of the spray arms.

WO-A-91/00744 discloses a fragranced article for use in fragrancing heated environments which article comprises a body of an ethylene vinyl acetate copolymer together with a fragrancing substance. This article finds particular application in fragrancing dishwashers. The tag can be demountably retained on a structure, for instance on a strut of a dishwasher rack. A disadvantage of these articles is that the fragrancing effect is a-specific and may be present in the entire washing area.

### Summary of the Invention

The invention provides a method for inhibiting malodour generated from the interior of an automatic washing machine, in particular an automatic dishwasher, the machine having a wash chamber closable by a hinged door, and the method comprising the step of positioning a freshening strip in the machine, wherein the strip is positioned on an interior surface of the door which is exposed to the flow of steam exiting from the wash chamber as the door is opened upon completion or interruption of a wash cycle.

The invention also provides an automatic washing machine, in particular an automatic dishwasher, the machine having a wash chamber closable by a hinged door, in which a freshening strip is positioned in the machine, wherein the strip is positioned on an interior surface of the door which is exposed to the flow of steam exiting from the wash chamber as the door is opened upon completion or interruption of a wash cycle.

The invention enables the air coming from the machine directly after the wash to be freshened without the disadvantages associated with prior art fragrancing methods such as those described above.

### Description of the Preferred Embodiments

The invention will be further described below with particular reference to automatic dishwashers, but could also be adapted for use with other automatic washing machines having a wash chamber closable by a hinged door, such as washing machines for laundry.

The freshening strip is preferably positioned on an interior surface of the door which is shielded from the interior environment of the wash chamber when the door is in the closed position.

This prevents premature degradation of the freshening strip resulting from exposure to the high temperatures and harsh chemicals encountered in the interior environment of the wash chamber when the wash cycle is in operation.

In a typical configuration, an automatic dishwasher comprises a front loading door hinged at the bottom to a wash chamber which is fitted beneath a work surface or another domestic appliance such as an electric or microwave oven. The overlying work surface or where applicable, domestic appliance, projects relative to the wash chamber by a distance at least equal to the thickness of the front door.

When the front loading door is closed, the upper edge of the door is hidden and protected by the work surface or domestic appliance.

The front portion of the door is usually covered by a painted material such as a plastics laminate, and the door is closed at the rear by an inner panel (typically metallic) which connects sealingly with the edges of the loading opening of the wash chamber. Typically a compressible and resilient seal, such as a rubber gasket, is disposed around the edges of the loading opening of the wash chamber to seal the inner panel of the door relative to the wash chamber when the door is in the closed position, and the inner panel is provided with a latching mechanism which prevents the opening of the door and the operation of the dishwasher unless the door is latched in the closed position.

Preferably the freshening strip is located on the upper peripheral wall of the inner panel.

Most preferably the freshening strip is located on the upper peripheral wall of the inner panel so that it is spaced apart from the region of the door seal when the door is in the closed position, and also spaced apart from the region of the door latching mechanism. This avoids premature degradation of the strip and leakage via the door seal.

The freshening strip may be secured in position by means of double-sided adhesive tape, or alternatively the freshening strip may include an adhesive backing paper for this purpose.

The freshening strip is typically polygonal in shape, and preferably substantially rectangular in shape. This shape enables the width of the strip to be selected so that it can be located on the upper peripheral wall of the inner panel as described above without impinging upon the region of the door seal when the door is in the closed position. Also the depth of the strip may be selected so that it does not impede door closure.

Most preferably the freshening strip is of substantially rectangular shape with a width suitably ranging from 0.5 to 3, preferably from 1 to 2 cm, a length suitably ranging from 1 to 20, preferably from 2 to 5 cm and a depth
suitably ranging from 0.05 to 0.4, preferably from 0.1 to 0.2 cm.

The freshening strip is suitably formed from a perfumed gel, which gives controlled release of perfume according to the moisture and/or temperature of its surrounding environment.

Optionally other forms than a gel are used, such as embedded/pressed particles or beads in other materials.

The weight of perfumed gel in the freshening strip (prior to use) suitably ranges from 0.2 to 3 grams, preferably from 0.5 to 2 grams, most preferably from 1 to 2 grams.
Typically the perfumed gel contains from 15% to 70% by weight perfume (by weight based on the total weight of the gel).

Preferably the perfumed gel contains from 20% to 60%, more preferably from 35 to 40% by weight perfume (by weight based on the total weight of the gel).

Preferably the perfumed gel is formed by dissolving a gelling agent in the perfume.

Suitable methods of forming the perfumed gel include heating, melting and mixing the perfume and gel together in a vessel, or alternatively, heating and melting the gel in a vessel, followed by adding the perfume to the melted gel and then mixing.

The perfume will typically be a derivative of a liquid hydrocarbon. It may be a discrete chemical but more typically will be a complex blend of volatile liquid ingredients of natural or synthetic origin.

Suitable gelling agents may be selected by those skilled in the art, and include absorbents, starch based systems, modified celluloses, natural gums and other materials which can form a gel when mixed with the perfume.

Preferred gelling agents used to form the perfumed gel have a melting point of at least 70 degrees C, more preferably at least 75 degrees C, most preferably at least 80 degrees C, so that the perfumed gel is able to withstand the temperature of steam exiting from the wash chamber as the door is opened upon completion or interruption of a wash cycle.

The gelling agent will typically be slightly water-soluble so that a dose of perfume is released upon exposure to the flow of steam. This enables controlled release of perfume over multiple dishwashing cycles. By "multiple dishwashing cycles" is meant at least two dishwashing cycles.
Preferably the controlled release may occur over a period of 10 to 100, more preferably 20 to 50, dishwashing cycles. By "dishwashing cycle" in this instance is meant a complete dishwashing cycle, including any pre wash, a wash at normal temperature (e.g. 50°C), and any rinses.

Suitable gelling agents used to form the perfumed gel include polyamide resins, and in particular polymerised fatty acid-based polyamide resins (i.e. polyamide resins which are prepared, in part, from polymerised fatty acid or reactive equivalents thereof). These materials create clear gels in a wide variety of solvents and are described for example in WO03/050164. Commercially available examples of these materials are the SYLVAGEL™ series of gelling agents from Arizona Chemicals, and a preferred example is SYLVAGEL™ 1000.

The perfumed gel may include other optional additives as commonly used in the art, including inert additives such as pigmented or pearlescent particles for aesthetic purposes, or soluble additives such as colorants. Such materials could serve as an end-of-life indicator, by providing a cue to the user that the freshening strip requires replacing.

The freshening strip may be supplied to the consumer as a single unit, or alternatively in the form of a package containing a plurality of freshening strips. The latter may be advantageous since it allows the consumer to use a single freshening strip or alternatively a plurality of strips at any one time according to the desired perfume intensity. Also, freshening strips with different perfumes may be provided, which the consumer can use either singly or in combination to produce a desired overall fragrancing effect.

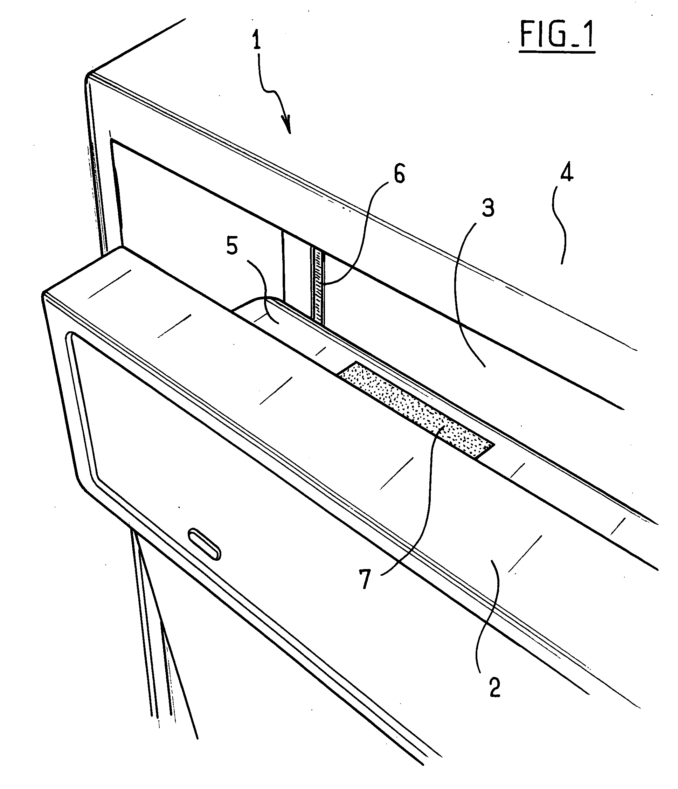

The preferred embodiment of the invention will be further illustrated with particular reference to FIGURE 1.

FIGURE 1 is a photograph of an automatic dishwasher with the door in partially open position, to show the location of the freshening strip according to the invention.

Referring to FIGURE 1, an automatic dishwasher 1 comprises a front loading door 2 hinged at the bottom to a wash chamber 3 which is fitted beneath a work surface 4. The front portion of door 2 is covered by a plastics laminate and the door 2 is closed at the rear by a metallic inner panel 5 which, when door 2 is closed, connects sealingly with the edges of the loading opening of the wash chamber 3 by means of rubber sealing gasket 6. Metallic inner panel 5 is provided with a latching mechanism (not shown) which prevents the opening of door 2 and the operation of the dishwasher 1 unless door 2 is latched in the closed position.

Freshening strip 7 is located on the upper peripheral wall of metallic inner panel 5, so that its edges are spaced apart from rubber sealing gasket 6 when door 2 is in the closed position, and spaced apart from the latching mechanism of metallic inner panel 5.

Freshening strip 7 is a rectangular strip of perfumed gel, with starting weight 1.4 g. The perfumed gel was made by gently heating and mixing perfume together with the gelling agent SYLVAGEL™ 1000 from Arizona Chemicals. The resultant perfumed gel contained 35 to 40 wt. perfume (by weight based on the total weight of the perfumed gel).

Freshening strip 7 is secured to metallic inner panel 5 by means of double sided adhesive tape (not shown).

Freshening strip 7 delivers a fragrancing effect through release of perfume from the perfumed gel when strip 7 is exposed to the flow of steam exiting from the wash chamber 3 when door 2 is opened.

In the context of this specification and claims, the word "steam" is used to indicate a water vapour or any similar vapour which may be hot or at a lower temperature.

Tests showed that this effect was sustained over at least 20 wash cycles, with an perfume intensity intermediate between that observed with the use of perfumed dishwasher detergent alone, and that observed with the use of a typical commercially available machine fragrancing device designed to be hung inside the dishwasher.

## Claims

1. A method for inhibiting malodour generated from the interior of an automatic washing machine, in particular an automatic dishwasher, the machine having a wash chamber (3) closable by a hinged door (2), and the method comprising the step of positioning a freshening strip (7) in the machine, **characterised in that** the strip (7) is positioned on an interior surface (5) of the door (2) which is exposed to the flow of steam exiting from the wash chamber (3) as the door (2) is opened upon completion or interruption of a wash cycle.

2. A method according to claim 1, in which the freshening strip (7) is positioned on an interior surface (5) of the door (2) which is shielded from the interior environment of the wash chamber (3) when the door (2) is in the closed position.

3. A method according to claim 2, in which the washing machine is an automatic dishwasher (1) in which the door (2) is closed at the rear by an inner panel which connects sealingly with the edges of the loading opening of the wash chamber (3), and the freshening strip (7) is located on the upper peripheral wall of the inner panel.

4. A method according to any one of claims 1 to 3, in which the freshening strip (7) is substantially rectangular in shape.

5. A method according to any one of claims 1 to 4, in which the freshening strip (7) is formed from a perfumed gel.

6. A method according to claim 5, in which the perfumed gel is made from polymerised fatty acid-based polyamide resin.

7. An automatic washing machine, in particular an automatic dishwasher (1), the machine having a wash chamber (3) closable by a hinged door (2), in which a freshening strip (7) is positioned in the machine, **characterised in that** the strip (7) is positioned on an interior surface (5) of the door (2) which is exposed to the flow of steam exiting from the wash chamber (3) as the door (2) is opened upon completion or interruption of a wash cycle.
